# FASCICULE DE BREVET EUROPEEN

(11) **EP 4 537 880 B1**
(45) Date de publication et mention de la délivrance du brevet: **25.02.2026**
(21) Numéro de dépôt: 24201586.5
(22) Date de dépôt: 20.09.2024
(51) Int. Cl.: A61M 16/00, A61M 16/10, A61M 16/12, G16H 20/40, G16H 40/63

(54) **AFFICHAGE D'UNE DOSE DE NO DE DÉPART PRÉFIXÉE SUR UN APPAREIL DE FOURNITURE DE GAZ MÉDICAL**
ANZEIGE EINER VOREINGESTELLTEN START-DOSIS VON NO AUF EINEM MEDIZINISCHEN GASBEREITSTELLUNGSGERÄT
DISPLAY OF A PRESET NO STARTING DOSE ON A MEDICAL GAS DELIVERY APPARATUS

(30) Priorité: 12.10.2023 FR 2310973
(43) Date de publication de la demande: 16.04.2025
(73) Titulaire: INOSYSTEMS, 92160 Antony (FR)
(72) Inventeur: BLANDIN, Yann, 92160 Antony (FR); MARCHAL, Frederic, 92160 Antony (FR); SCHMITT, Mary, 92160 Antony (FR); BOULANGER, Thierry, 92160 Antony (FR)
(74) Mandataire: Air Liquide

(56) Documents cités:
- EP-A1- 3 308 820
- EP-A1- 3 906 955
- EP-A1- 4 295 882
- FR-A1- 3 133 318
- US-A1- 2017 095 634
- US-A1- 2021 268 221
- US-A1- 2023 270 960

## Description

L'invention concerne un appareil ou dispositif de fourniture et monitorage d'un gaz médical contenant du monoxyde d'azote (NO), en particulier d'un mélange gazeux NO/N₂, couramment appelé appareil de fourniture de NO, utilisé pour traiter une ou des personnes souffrant d'hypertension artérielle pulmonaire aiguë, configuré pour afficher, lors de la mise en route de l'appareil, une dose de NO mémorisée dite « par défaut » choisie par le personnel médical et préenregistrée, aussi appelée dose de « départ ».

Le monoxyde d'azote inhalé (NOi) est un traitement de référence pour traiter les personnes, i.e. les patients, souffrant d'hypertension artérielle pulmonaire aiguë. Lorsqu'il est inhalé, le NO dilate les vaisseaux pulmonaires et augmente l'oxygénation en améliorant les échanges gazeux. Ces propriétés sont utilisées pour traiter différentes conditions médicales, comme l'Hypertension Artérielle Pulmonaire du Nouveau-né ou PPHN (pour *Persistent Pulmonary Hypertension of the Newborn*), le Syndrome de Détresse Respiratoire Aigüe ou SDRA observé principalement chez l'adulte ou les hypertensions pulmonaires (HP) en chirurgie cardiaque chez l'adulte ou l'enfant, comme décrit notamment par EP-A-560928, EP-A-1516639 et US-A-10,201,564.

La mise en œuvre d'un traitement par NOi comprend habituellement une ou des bouteilles de mélange NO/N₂ (ou un générateur de NO), un appareil de fourniture et monitorage de NO, un ventilateur médical et un kit patient comprenant un circuit patient et une interface respiratoire, telle une sonde d'intubation trachéale..., voire d'autres éléments, tel un humidificateur de gaz ou autre.

Usuellement, une faible quantité de NO gazeux (i.e. quelques ppm vol.), dilué dans de l'azote (N₂) est injectée et diluée dans un flux gazeux contenant de l'oxygène, typiquement au moins 20%vol. environ d'oxygène (O₂), tel un mélange N₂/O₂ ou de l'air, voire de l'oxygène pur, qui est véhiculé par le circuit patient d'une installation de fourniture de gaz, et le mélange gazeux final obtenu contenant du NO et de l'oxygène est ensuite inhalé par le patient.

La concentration ou dose de NO finale, qui correspond à une posologie, est déterminée par le médecin ou analogue, ou fixée par un protocole médical validé par l'équipe médicale. En général, elle est comprise entre 1 et 80 ppm en volume (ppmv), typiquement de l'ordre de 10 à 20 ppmv, en fonction de la population traitée, i.e. nouveau-nés, enfants, adolescents ou adultes, et de la maladie à traiter, dans le mélange gazeux final NO/N₂/O₂ administré par inhalation au patient considéré, c'est-à-dire après injection du mélange NO/N₂ dans le flux de gaz contenant de l'oxygène (i.e. environ >20 %vol.) véhiculé par le circuit patient.

La concentration ou dose de NO désirée est généralement réglée, ajustée ou sélectionnée par le personnel soignant, tel un médecin, une infirmière ou analogue, au début de la procédure de traitement d'un patient et dans l'urgence de la mise en œuvre du traitement, c'est-à-dire après mise en route de l'appareil de fourniture de NO.

Des exemples d'appareils de fourniture de NO permettant de régler la dose de NO à délivrer aux patients sont décrits par EP4295882, FR3133318, US2023/270960 et US2021/268221. Le document EP4295882 relève de l'article 54(3) CBE.

Pour ce faire, l'appareil de fourniture de NO affiche une dose ou concentration dite « de départ » nulle, c'est-à-dire une concentration de NO de 0 ppm, et le personnel soignant incrémente la dose par pallier ou entre/sélectionne directement une dose « de départ » désirée, par exemple 10, 15 ou 20 ppm, qui sera utilisée ensuite par l'appareil de fourniture de NO pour fournir un débit de NO adapté, i.e. un débit de mélange gazeux NO/N₂, permettant d'obtenir cette dose « de départ » réglée dans le mélange gazeux final administré par inhalation au patient à traiter.

Cependant, en pratique, des erreurs de dosage de NO surviennent parfois du fait d'erreurs de réglage ou fixation de la dose car le personnel soignant doit agir dans l'urgence, lors d'un démarrage de traitement par NO, et peut dès lors se tromper en entrant ou sélectionnant la dose de NO à administrer au départ.

Or, on comprend qu'une erreur de dose peut engendrer un risque pour le patient qui ne reçoit pas la posologie désirée, c'est-à-dire une concentration ou dose adaptée, en particulier un risque de surdosage si la teneur entrée est trop élevée ou, dans le cas contraire, un sous-dosage pouvant conduire alors à une absence d'effet thérapeutique du fait d'une dose insuffisante et non-efficace.

Un problème est donc de pouvoir accroître la sécurité d'un traitement par NO en évitant les erreurs de réglage de dose de NO pouvant se produire au début du traitement, c'est-à-dire les erreurs liées à la dose de NO de « départ ».

Une solution de l'invention concerne un appareil ou dispositif de fourniture d'un gaz contenant du NO utilisable pour traiter un patient, tel un mélange gazeux NO/N₂, comprenant :
- au moins un passage interne pour acheminer un flux de gaz contenant du NO,
- des moyens à valve agencés sur ledit au moins un passage interne,
- un afficheur graphique, et
- des moyens de pilotage à microprocesseur coopérant au moins avec les moyens à valve pour contrôler la fourniture de gaz, en particulier le débit de gaz dans au moins une partie dudit passage interne.

De plus, l'appareil ou dispositif de l'invention comprend :
- des moyens de mémorisation pour mémoriser une dose (i.e. une concentration) non-nulle de NO préfixée (NO_{mém}) correspondant à une concentration de NO dite de départ à administrer par inhalation à un patient au démarrage d'un traitement par NO inhalé,
- les moyens de pilotage sont configurés pour commander un affichage sur l'afficheur graphique de la dose de NO préfixée (NO_{mém}) ayant été mémorisée,
- et l'afficheur graphique est configuré pour opérer, dès une mise en route ou en service de l'appareil et avant tout démarrage du traitement par NO inhalé, un affichage par défaut de ladite dose de NO préfixée (NO_{mém}) ayant été mémorisée.

Autrement dit, selon l'invention, l'appareil est configuré pour afficher lors de la mise en route ou en service de l'appareil, c'est-à-dire dès son allumage par actionnement par exemple d'une touche ou d'un bouton de mise en route (On/Off), une dose non-nulle de NO par défaut mémorisée correspondant à la concentration de NO non-nulle qui sera administrée au patient au commencement de son traitement, typiquement après appui de l'utilisateur, i.e. personnel soignant, tel que médecin ou analogue, d'une touche de début de traitement, typiquement une touche virtuelle s'affichant sur l'écran d'affichage de l'appareil. Ceci évite au personnel soignant de devoir entrer cette dose et limite donc les risques d'erreurs et les inconvénients susmentionnés.

Selon le mode de réalisation considéré, l'appareil de l'invention peut comprendre l'une ou plusieurs des caractéristiques suivantes :
- la dose de NO préfixée (NO_{mém}) mémorisée est la seule dose de NO affichée au démarrage de l'appareil (i.e. lors de sa mise en route), c'est-à-dire qu'elle s'affiche sur l'écran d'affichage de manière automatique et sans alternative possible, i.e. avant tout démarrage d'un traitement par iNO.
- la dose de NO préfixée (NO_{mém}) mémorisée est affichée avant tout commencement d'administration de NO au patient.
- la dose de NO préfixée (NO_{mém}) mémorisée est affichée au moment de la mise en route ou en service de l'appareil mais avant tout début d'administration de NO par l'appareil.
- la dose de NO préfixée (NO_{mém}) mémorisée est affichée immédiatement au moment de la mise en route ou en service de l'appareil.
- la dose de NO préfixée (NO_{mém}) mémorisée n'est pas un seuil d'alarme.
- la dose de NO préfixée (NO_{mém}) correspond à la concentration de NO désirée dans le mélange gazeux à administrer au patient, i.e. la posologie à observer dans le cadre d'un traitement avec fourniture de NO inhalé audit patient. Typiquement, le mélange gazeux administré, aussi appelé mélange final, contient du NO, de l'azote (N₂) et de l'oxygène (O₂), voire d'autres composés, telle de la vapeur d'eau et/ou des impuretés non-souhaitées comme le NO₂.
- les moyens de pilotage sont configurés pour commander un affichage par défaut de la dose de NO préfixée (NO_{mém}) mémorisée dès mise en service de l'appareil, i.e. dès mise en route, par exemple dès appui sur un bouton ou une touche d'allumage/extinction de l'appareil (i.e. touche de « on/off » en anglais) et éventuellement après un temps de latence nécessaire à l'initialisation de l'appareil ou analogue, notamment après un autotest de démarrage visant à s'assurer que les principaux éléments de l'appareil sont fonctionnels, par exemple le ou les processeurs, le système d'exploitation etc...
- les moyens de pilotage sont configurés pour commander un affichage par défaut de la dose de NO préfixée (NO_{mém}) mémorisée, après mise en service de l'appareil, i.e. dès mise en route, et avant tout démarrage effectif du traitement du patient avec administration de gaz contenant du NO.
- les moyens de pilotage sont configurés pour commander un affichage par défaut de la dose de NO préfixée (NO_{mém}) mémorisée, après mise en service de l'appareil, i.e. dès mise en route, et avant toute injection de gaz contenant du NO dans la branche inspiratoire du circuit patient.
- les moyens de pilotage sont configurés pour commander un affichage automatique et par défaut de la dose de NO préfixée (NO_{mém}) mémorisée dès mise en route de l'appareil et de l'afficheur graphique.
- les moyens de pilotage sont configurés pour commander un affichage de la dose de NO préfixée (NO_{mém}) dans un espace dédié de l'écran, typiquement une fenêtre d'affichage.
- les moyens de pilotage sont configurés pour contrôler tous les affichages opérés sur l'afficheur graphique.
- la dose de NO préfixée (NO_{mém}) est comprise entre 1 et 40 ppmv, de préférence entre 5 et 20 ppmv.
- la dose de NO préfixée (NO_{mém}) est typiquement une dose de 5, 10, 15 ou 20 ppmv.
- les moyens de mémorisation sont configurés pour mémoriser une dose de NO préfixée (NO_{mém}) comprise entre 1 et 40 ppmv, de préférence entre 5 et 20 ppmv, typiquement 5, 10, 15 ou 20 ppmv.
- il comprend en outre une touche (de validation) de début de traitement, c'est-à-dire une touche de sélection, actionnable (i.e. sélectionnable) par l'utilisateur, dont l'actionnement ou la sélection engendre une fourniture d'un flux (i.e. débit) de gaz contenant du NO permettant d'obtenir la dose de NO préfixée mémorisée (NO_{mém}).
- ledit au moins un passage interne servant à acheminer le flux de gaz comprend un ou plusieurs conduits de gaz, passages, tuyaux ou analogues.
- le (au moins un) passage interne est configuré pour acheminer le flux de gaz contenant du NO entre (au moins) une entrée de gaz et (au moins) une sortie de gaz fournissant le gaz contenant le NO, i.e. un mélange NO/N₂.
- les moyens à valve comprend une ou des valves ou vannes, en particulier une ou des électrovannes ou une ou des vannes tout ou rien.
- l'afficheur graphique est à dalle tactile.
- l'afficheur graphique tactile fait partie d'une IGU ou interface graphique utilisateur.
- l'afficheur graphique comprend un écran en couleurs ou en noir et blanc.
- les moyens de pilotage comprennent un ou plusieurs microprocesseurs.
- le ou les microprocesseurs sont agencés sur une carte électronique.
- les moyens de pilotage coopèrent avec les moyens à valve pour contrôler la fourniture de gaz, en particulier le débit de gaz contenant du NO, en fonction de la dose de NO à fournir, c'est-à-dire la dose de NO préfixée (NO_{mém}).
- les moyens de pilotage coopèrent avec les moyens à valve pour autoriser une fourniture de gaz, en particulier d'un débit de gaz contenant du NO, au démarrage d'un traitement du patient.
- à l'inverse, les moyens de pilotage coopèrent avec les moyens à valve pour empêcher ou interdire une fourniture de gaz, en particulier d'un débit de gaz contenant du NO, avant le démarrage d'un traitement du patient, en particulier avant appui par l'utilisateur sur une touche de début de traitement.
- l'afficheur graphique est configuré pour afficher des informations, notamment la dose de NO à fournir, c'est-à-dire la dose de NO préfixée (NO_{mém}).
- les moyens de pilotage sont configurés pour commander l'affichage sur l'afficheur graphique de la dose de NO exprimée en ppmv.
- l'afficheur graphique est configuré pour afficher en outre une touche de début de traitement.
- la touche de début de traitement est une touche virtuelle affichée sur l'afficheur graphique.
- l'afficheur graphique à dalle tactile.
- l'afficheur graphique est configuré pour détecter le contact d'un doigt d'un utilisateur, notamment de son index, appuyant sur la surface de l'afficheur graphique, en particulier sur la dalle tactile de l'afficheur graphique.
- les moyens de pilotage sont en outre configurés pour commander un affichage sur l'afficheur graphique d'au moins une touche ou fenêtre de confirmation, c'est-à-dire une touche virtuelle, en réponse à un actionnement par l'utilisateur de la touche de début de traitement (i.e. de la touche de validation).
- les moyens de pilotage sont configurés pour commander les moyens à valve pour fournir un débit donné de gaz contenant du NO permettant d'obtenir la dose de NO préfixée mémorisée (NO_{mém}), en réponse à un actionnement par l'utilisateur d'au moins la touche de début de traitement, en particulier d'au moins la touche virtuelle (de validation) de début de traitement affichée sur l'afficheur graphique.
- les moyens de pilotage sont configurés pour commander les moyens à valve pour fournir un débit donné de gaz contenant du NO permettant d'obtenir la dose de NO préfixée mémorisée (NO_{mém}), en réponse à des actionnements successifs par l'utilisateur de la touche de début de traitement puis de la touche de confirmation, typiquement des touches/zones de début de traitement et de confirmation virtuelles, c'est-à-dire que les moyens de pilotage commandent les moyens à valve pour délivrer du gaz uniquement après sélection (e.g. appui sur) par l'utilisateur de la touche de début (i.e. de vaidation) de traitement et ensuite confirmation (e.g. appui sur) de la touche ou zone de confirmation de manière à confirmer la dose de NO et/ou le début du traitement.
- les moyens de pilotage sont configurés pour commander un affichage de la touche de confirmation au sein d'une fenêtre d'affichage affichée sur l'écran d'affichage.
- à l'inverse, les moyens de pilotage sont configurés pour commander les moyens à valve de sorte de ne pas fournir de flux de gaz contenant du NO, en réponse à un actionnement par l'utilisateur d'au moins une touche ou zone d'annulation de traitement, en particulier une touche ou zone virtuelle affichée sur l'afficheur graphique, c'est-à-dire qu'une touche ou zone permettant de refuser, annuler ou analogue tout traitement du patient.
- l'écran est configuré pour afficher une première touche ou zone de confirmation dont la sélection par l'utilisateur (e.g. par appui digital) permet de confirmer la dose de départ proposée et lancer le traitement, par exemple une touche ou zone appelée « OK », « GO », « Début » ou analogue.
- l'écran est configuré pour afficher une seconde touche ou zone de confirmation dont la sélection par l'utilisateur (e.g. par appui digital) permet d'annuler ou stopper tout démarrage de traitement avec fourniture de NO, par exemple une touche ou zone appelée « Stop », « Abandon », « Arrêt » ou similaire.
- selon un mode de réalisation, l'écran est configuré pour afficher la seconde touche ou zone de confirmation en réponse à un appui par l'utilisateur sur la première touche ou zone de confirmation.
- selon un mode de réalisation, l'écran est configuré pour afficher les deux touches de confirmation de manière successive, c'est-à-dire l'une après l'autre.
- selon un mode de réalisation, l'écran est configuré pour afficher les deux touches de confirmation de manière simultanée, par exemple juxtaposées.
- selon un mode de réalisation, l'écran est configuré pour afficher une fenêtre de confirmation comprenant les deux touches ou zones de confirmation distinctes, par exemple une fenêtre de confirmation divisée en deux zones distinctes, i.e. juxtaposées.
- selon un mode de réalisation, l'écran est configuré pour afficher la fenêtre de confirmation de manière superposée à la fenêtre d'affichage de dose de NO de départ et à la touche de validation de début de traitement, en les masquant partiellement ou totalement.
- l'afficheur graphique est en outre configuré pour afficher simultanément à la fenêtre de confirmation, une phrase ou analogue rappelant à l'utilisateur ce qu'il doit confirmer ou infirmer, par exemple : *Démarrage du traitement à la dose de 10 ppm ?* ou d'autres informations utiles.
- la fenêtre de confirmation comprend des touches ou zones de confirmation tactiles, i.e. virtuelles venant s'afficher sur la dalle de l'écran tactile.
- les moyens de pilotage agissent en réponse à tout appui digital d'un utilisateur sur l'une et/ou l'autre des touches ou zones virtuelles affichées sur l'écran d'affichage à dalle tactile.
- le ou les actionnements ou sélections par l'utilisateur de la touche de début de traitement et/ou de la ou des touches ou zones de confirmation se font par appui digital de l'utilisateur, par exemple un appui de l'index.
- les moyens de pilotage sont configurés pour commander un affichage simultané, sur l'afficheur graphique à dalle tactile, de la dose de NO préfixée (NO_{mém}) et de la touche de début de traitement, i.e. la touche virtuelle permettant de démarrer/commencer un traitement du patient.
- l'afficheur graphique à dalle tactile est configuré pour afficher la dose de NO préfixée (NO_{mém}) et la touche de début de traitement de manière juxtaposée, c'est-à-dire l'une à côté de l'autre.
- les moyens de pilotage sont configurés pour commander les moyens à valve pour délivrer un débit de gaz donné correspondant à la dose de NO préfixée (NO_{mém}) comprise entre 5 et 40 ppmv, de préférence entre 5 et 20 ppmv.
- les moyens de mémorisation comprennent un dispositif de mémorisation informatique, telle une mémoire informatique, par exemple une mémoire flash.
- la mémoire informatique est agencée sur la carte électronique.
- il comprend des moyens d'alimentation électrique, telle une liaison électrique au secteur (110/220V).
- il comprend un boitier rigide, c'est-à-dire une carcasse extérieure de protection.
- le boitier rigide comprend ledit au moins un passage interne, lesdits moyens à valve, l'afficheur graphique et les moyens de pilotage.
- l'appareil est configuré pour opérer un monitorage et une fourniture de NO gazeux, i.e. de gaz contenant du NO, tel un mélange NO/N₂.
- la touche de début de traitement, typiquement une touche de sélection virtuelle, coopère avec les moyens de pilotage pour leur fournir au moins un signal de commande pour opérer une fourniture d'un débit de gaz contenant du NO correspondant à la dose de NO préfixée mémorisée (NO_{mém}) désirée dans le mélange gazeux final.
- les moyens de pilotage sont configurés pour commander les moyens à valves en réponse audit signal de commande.
- il comprend des moyens de réglage de dose, notamment des moyens de configuration, permettant d'ajuster ou modifier la dose de NO affichée par défaut, par exemple permettant d'augmenter ou diminuer la dose de NO préfixée mémorisée (NO_{mém}) affichée sur l'écran d'affichage.
- il comprend des moyens de configuration permettent de régler ou sélectionner et mémoriser la dose de NO désirée (NO_{mém}).
- les moyens de configuration comprennent un menu de configuration accessible via l'écran d'affichage.
- les moyens de réglage de dose comprennent une ou des touches de réglage, par exemple des touches « + » et « - » permettant d'incrémenter ou de décrémenter de 1 ppm (ou plus) la dose affichée.
- la ou les touches de réglage sont des touches virtuelles s'affichant sur l'écran d'affichage, i.e. écran à dalle tactile.
- la ou les touches de réglage sont des touches virtuelles configurées pour engendrer une augmentation ou diminution de la dose affichée en réponse à un appui de l'utilisateur sur l'une ou l'autre de ces touches.
- les moyens de réglage de dose coopèrent avec les moyens de pilotage.
- les moyens de pilotage modifie la valeur de dose de NO affichée en réponse à une activation par l'utilisateur des moyens de réglage de dose, en particulier en réponse à un appui digital sur l'une ou l'autre de ces touches.

L'invention porte aussi sur une installation d'administration de gaz à un patient, i.e. d'un gaz contenant du NO, comprenant :
- au moins une source de gaz contenant du NO gazeux, en particulier un mélange gazeux NO/N₂,
- un appareil de fourniture de gaz selon l'invention, en particulier comme décrit ci-avant, alimenté en gaz contenant du NO par ladite au moins une source de gaz, tel le mélange gazeux NO/N₂,
- un ventilateur médical pour fournir un gaz contenant de l'oxygène, tel de l'air ou un mélange gazeux O₂/N₂, et
- une ligne d'alimentation alimentée par l'appareil de fourniture de gaz en gaz contenant du NO, tel le mélange gazeux NO/N₂, et par le ventilateur médical gaz contenant de l'oxygène, tel de l'air ou le mélange O₂/N₂.

Selon le mode de réalisation considéré, l'installation d'administration de gaz de l'invention peut comprendre l'une ou plusieurs des caractéristiques suivantes :
- le ventilateur médical, c'est-à-dire un appareil d'assistance ventilatoire, est en communication fluidique avec la ligne d'alimentation pour alimenter ladite ligne d'alimentation avec un gaz respiratoire contenant au moins environ 20% vol. d'oxygène, de préférence au moins environ 21% vol. d'oxygène, en particulier de l'air ou un mélange N₂/O₂.
- le ventilateur médical est un appareil d'assistance respiratoire fournissant le gaz à pression constante ou, alternativement, le ventilateur médical est un ventilateur de type HFO (*High Frequency Oscillations*) délivrant le gaz par oscillations à haute fréquence.
- la ligne d'alimentation en gaz est alimentée en mélange NO/N₂ par l'appareil de fourniture de gaz et en un gaz respiratoire contenant au moins environ 20% vol. d'oxygène, de préférence au moins environ 21% vol. d'oxygène, de préférence de l'air ou un mélange N₂/O₂, par le ventilateur médical.
- la ligne d'alimentation en gaz est alimentée en mélange NO/N₂ par l'appareil de fourniture de gaz et en un gaz respiratoire contenant de l'oxygène, de préférence de l'air ou un mélange N₂/O₂, par le ventilateur médical de manière à y former un mélange gazeux final à administrer au patient contenant du NO, de l'azote et de l'oxygène, voire d'autres composés comme de la vapeur d'eau et/ou des impuretés, comme l'argon ou des espèces NO₂ formées par oxydation d'une partie du NO.
- le mélange gazeux final à administrer au patient contient de l'azote, de l'oxygène et du NO en une proportion correspondant à la dose de NO préfixée (NO_{mém}).
- le mélange gazeux final à administrer au patient contient au moins 20%vol. d'oxygène, du NO en une proportion (approximativement) égale à la dose de NO préfixée (NO_{mém}) et de l'azote.
- la (les) source de gaz contient un mélange gazeux NO/N₂ contenant moins de 2000 ppmv de NO, le reste étant de l'azote, de préférence moins de 1000 ppmv de NO, le reste étant de l'azote, préférentiellement, la source de gaz thérapeutique contient un mélange NO/N₂ contenant de 250 à 900 ppmv de NO, le reste étant de l'azote, par exemple de l'ordre de 800 ppmv de NO, le reste étant de l'azote.
- elle comprend en outre un humidificateur de gaz agencé sur la ligne d'alimentation en gaz, de préférence en aval du site où le dispositif de fourniture de gaz thérapeutique est raccordé fluidiquement à ladite ligne d'alimentation en gaz de manière à l'alimenter en gaz thérapeutique.
- elle comprend en outre une ligne de récupération des gaz expirés par le patient.
- la ligne d'alimentation en gaz et la ligne de récupération des gaz expirés sont raccordées à une pièce de raccordement, de préférence une pièce en Y, et définissent ou forment tout ou partie d'un circuit patient.
- la ligne d'alimentation forme une branche inspiratoire du circuit patient.
- la ligne de récupération des gaz expirés forme une branche expiratoire du circuit patient.
- l'appareil de fourniture de NO comprend en outre une ligne d'analyse de gaz reliée fluidiquement à la ligne d'alimentation en gaz, i.e. la branche inspiratoire.
- la ligne d'alimentation en gaz, i.e. la branche inspiratoire, comprend un capteur de débit agencé entre le ventilateur et le site d'injection du gaz contenant le NO provenant de l'appareil de fourniture de NO.
- le capteur de débit est relié aux moyens de pilotage de l'appareil de fourniture de NO.
- le capteur de débit mesure le débit de gaz contenant de l'oxygène délivré par le ventilateur médical et circulant dans la ligne d'alimentation en gaz, i.e. la branche inspiratoire.
- le capteur de débit retourne des valeurs ou des signaux de débit de gaz
- les moyens de pilotage sont configurés pour contrôler les moyens à valve pour délivrer le flux gazeux contenant du NO (e.g. mélange NO/N₂) à un débit donné permettant d'obtenir une dose de NO dans la ligne d'alimentation en gaz correspondant à la dose mémorisée (NO_{mém}), lequel débit donné est déterminé à partir du débit de gaz (e.g. d'air) mesuré par le capteur de débit et de la concentration en NO dans le mélange NO/N₂.
- la ligne d'alimentation en gaz alimente une interface respiratoire, par exemple un masque respiratoire, une sonde d'intubation trachéale ou analogue.
- la ligne d'alimentation en gaz, i.e. la branche inspiratoire, est raccordée fluidiquement à un port de sortie du ventilateur médical de manière à récupérer et acheminer le gaz délivré par le ventilateur médical.
- la ligne de récupération des gaz expirés, i.e. la branche expiratoire, est raccordée fluidiquement à un port d'entrée du ventilateur médical de manière à acheminer jusqu'au ventilateur médical tout ou partie des gaz expirés par le patient.
- au moins une source de gaz thérapeutique comprend un ou plusieurs récipients de gaz, en particulier une ou des bouteilles de gaz sous pression.
- le (ou les) récipient de gaz est équipé d'un robinet de distribution de gaz avec ou sans détenteur intégré (RDI).
- le robinet de distribution de gaz est en alliage de cuivre, tel du laiton, et/ou est équipé d'un capotage de protection agencé autour du robinet de distribution de gaz, par exemple en matériau polymère (i.e. plastique), en métal ou leurs combinaisons.
- le (ou les) récipient de fluide est (sont) une bouteille de gaz sous pression contenant, lorsqu'il est plein, un mélange gazeux, en particulier NO/N₂, à une pression d'au moins 150 à 200 bar abs, voire d'au moins 250 à 300 bar abs.
- le récipient de fluide a une forme générale cylindrique, en particulier d'ogive.

La présente divulgation porte en outre sur une méthode de traitement thérapeutique d'une personne humaine, i.e. d'un patient, mettant en œuvre un appareil de fourniture de NO et/ou d'une installation d'administration de gaz, i.e. d'un gaz contenant du NO, comprenant un appareil de fourniture de NO, pour fournir à ladite personne, un mélange gazeux contenant du NO à une posologie donnée, ladite personne inhalant ledit mélange gazeux, en particulier un mélange gazeux final à base de NO comprenant du NO, au moins 20% d'oxygène et de l'azote, de préférence au moins 21% d'oxygène.

Dans le cadre de cette méthode de traitement :
- la personne souffre d'hypertension artérielle pulmonaire aiguë, en particulier d'Hypertension Artérielle Pulmonaire du Nouveau-né ou PPHN, d'un Syndrome de Détresse Respiratoire Aigüe ou SDRA ou d'hypertensions pulmonaires (HP) lors d'une chirurgie cardiaque.
- la personne est un adulte, un adolescent, un enfant, un bébé, un nouveau-né ou un prématuré.
- le mélange gazeux final est administré au moyen d'une interface respiratoire, en particulier un masque respiratoire ou une sonde d'intubation trachéale.
- le mélange gazeux final contient de l'azote, de l'oxygène (>21% environ) et du NO en une proportion correspondant à la dose de NO préfixée (NO_{mém}).
- le mélange gazeux final contient moins de 80 ppmv de NO, typiquement entre 5 et 40 ppmv.
- le mélange gazeux final est administré pendant de 1 à plusieurs heures à 1 ou plusieurs jours.

D'une façon générale, dans le cadre de l'invention :
- « ppmv » signifie partie par million en volume,
- « %vol. » » signifie pourcentage en volume.
- « NO » désigne le monoxyde d'azote.
- « NO₂ » désigne le dioxyde d'azote.
- « N₂ » désigne l'azote.
- « O₂ » désigne l'oxygène.
- les termes « concentration », « dose » et « teneur » sont considérés comme équivalents et substituables.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens à valves » peuvent être remplacés par « dispositif à valves », les « moyens de mémorisation» peuvent être remplacés par « dispositif de mémorisation» ...

L'invention va maintenant être mieux comprise grâce à la description détaillée suivante, faite à titre illustratif mais non limitatif, en référence aux figures annexées parmi lesquelles :
Fig. 1 représente un mode de réalisation d'une installation d'administration de gaz, i.e. d'un gaz contenant du NO, comprenant un appareil de fourniture de NO selon l'invention.
Fig. 2 schématise le fonctionnement d'un appareil de fourniture de NO selon l'invention.
Fig. 3 schématise un mode de réalisation de l'affichage de la dose de NO de départ sur l'écran d'affichage d'un appareil de fourniture de NO selon l'invention, tel celui de Fig. 2, en particulier lorsqu'il est utilisé dans une installation d'administration selon Fig. 1, avant démarrage de toute fourniture de NO au patient.
Fig. 4 schématise un mode de réalisation du menu de configuration permettant de fixer la dose de NO de départ d'un appareil de fourniture de NO selon l'invention, tel celui de Fig. 2 et Fig. 3.

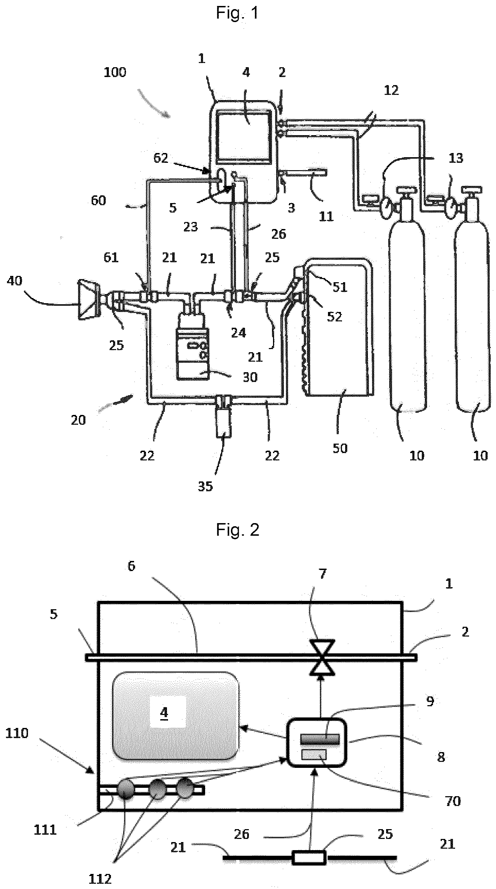

Un mode de réalisation d'une installation d'administration de gaz 100 est illustré sur Fig. 1, à savoir de fourniture d'un mélange thérapeutique à base de NO destiné à traitement un patient en ayant besoin.

L'installation 100 comprend ici deux bouteilles de gaz sous pression en tant que sources de gaz 10 contenant chacune un mélange gazeux NO/N₂ contenant jusqu'à 1% vol de NO, typiquement entre 100 et 1500 ppmv de NO (reste N₂), à savoir ici un mélange gazeux NO/N₂ contenant par exemple 450 ou 800 ppmv de NO (reste N₂), qui alimentent en mélange NO/N₂, un dispositif ou appareil de fourniture, i.e. de délivrance, de NO 1 permettant de monitorer/suivre et contrôler la fourniture du mélange gazeux NO/N₂.

Les bouteilles de gaz 10 sont reliées fluidiquement à l'appareil de fourniture de NO 1, via des lignes d'amenée de gaz 12, tel que des tuyaux ou conduits flexibles ou analogues, qui peuvent être équipées de dispositifs de régulation et/ou de suivi de la pression de gaz, tels que détendeur de gaz 13, manomètres... Les lignes d'amenée de gaz 12 sont reliées à une ou plusieurs entrées de gaz 2 du dispositif de délivrance de NO 1 qui alimentent un passage de gaz interne servant à acheminer le gaz au sein du dispositif de délivrance de NO 1, c'est-à-dire dans le boitier ou la carcasse externe du dispositif de délivrance de NO 1.

Le dispositif de fourniture de NO 1 comprend aussi une entrée d'oxygène 3 reliée fluidiquement, via une ligne d'amenée d'oxygène 12, tel un tuyau flexible ou analogue, à une source d'oxygène par exemple une bouteille d'oxygène sous pression ou un réseau hospitalier, c'est-à-dire une canalisation d'alimentation en oxygène agencée dans un établissement hospitalier.

L'installation d'administration de gaz 100 comprend par ailleurs un ventilateur médical 50, c'est-à-dire un appareil d'assistance respiratoire, qui fournit un flux de gaz respiratoire contenant de l'oxygène, typiquement de l'ordre d'au moins 20% environ d'oxygène, de préférence d'au moins 21% environ d'oxygène, tel de l'air ou un mélange oxygène/azote (N₂/O₂), voire de l'oxygène pur dans certains cas.

Le ventilateur médical 50 et l'appareil de fourniture de NO 1 de l'installation d'administration de gaz 100 sont en communication fluidique avec une ligne d'alimentation en gaz, aussi appelée branche inspiratoire 21, d'un circuit patient 20. La ligne d'alimentation en gaz 21 sert à acheminer le flux gazeux vers le patient, lequel est formé par mélange du flux à base d'oxygène (i.e. air ou mélange NO/N₂) provenant du ventilateur médical 50 et du flux contenant le NO, i.e. le mélange gazeux NO/N₂, délivré par le dispositif de fourniture de NO 1.

Plus précisément, le dispositif de fourniture de NO 1 délivre ou injecte un débit donné, i.e. contrôlé, de mélange gazeux NO/N₂, par exemple à 450 ou 800 ppmv de NO, dans la ligne d'alimentation en gaz 21, via un conduit ou ligne d'injection 23 venant se raccorder à une sortie 5 de NO de l'appareil 1, de manière à mélanger (en 24) le flux de NO/N₂ au flux de gaz à base d'oxygène (avec au moins environ 20 à 21% d'O₂), e.g. de l'air ou un mélange oxygène/azote, délivré par le ventilateur médical 50 et véhiculé par la branche inspiratoire 21 du circuit patient 20 de sorte d'obtenir un mélange final contenant essentiellement du NO à la posologie désirée, de l'azote (N₂) et de l'oxygène (O₂), et éventuellement des impuretés inévitables (e.g. argon, CO₂, NO₂, ....), c'est-à-dire un mélange gazeux NO/N₂/O₂.

La branche inspiratoire 21 comprend en outre un humidificateur de gaz 30 agencé en aval du site 24 où se fait l'injection de NO dans la branche inspiratoire 21. Il permet d'humidifier le flux de gaz, e.g. le mélange gazeux NO/N₂/O₂, avant qu'il ne soit inhalé par le patient à traiter, au moyen d'une interface respiratoire 40, telle une sonde d'intubation trachéale, un masque respiratoire ou analogue.

Est prévue aussi une ligne de récupération des gaz expirés par le patient formant la branche expiratoire 22 du circuit patient 20. La ligne d'alimentation en gaz ou branche inspiratoire 21 et la ligne de récupération ou branche expiratoire 22 des gaz expirés sont raccordées à une pièce de raccordement 25, de préférence une pièce en Y.

La branche inspiratoire 21 est raccordée, en amont, fluidiquement à un port de sortie 51 du ventilateur médical 50, tel un connecteur, raccord ou analogue, de manière à récupérer et acheminer le gaz à base d'oxygène, typiquement de l'air ou mélange N₂/O₂ (contenant environ >20 à 21% d'O₂) délivré par le ventilateur médical 50, alors que la branche expiratoire 22 véhiculant les gaz expirés est raccordée fluidiquement à un port d'entrée 52 du ventilateur médical 50, tel un connecteur, raccord ou analogue, de manière à retourner au ventilateur médical 50 tout ou partie du débit des gaz expirés par le patient.

La branche expiratoire 22 des gaz expirés peut comprendre un ou plusieurs composants optionnels, comme par exemple un dispositif d'élimination du CO₂ 35, i.e. un piège à CO₂, tel un bac à chaud ou autre, permettant d'éliminer le CO₂ présent dans les gaz expirés par le patient ou un filtre ou autre.

Un capteur de débit 25, par exemple du type à fil chaud ou à différentiel de pression, est agencé sur la ligne d'alimentation en gaz 21, entre le ventilateur 50 et l'humidificateur 30, et est relié au dispositif de délivrance de NO 1, via une ligne de mesure de débit 26. Cet agencement sert à mesurer le débit de gaz à base d'oxygène délivré par le ventilateur 50, e.g. air ou un mélange N₂/O₂, circulant dans la branche inspiratoire 21, en amont du site de 24 raccordement du conduit ou ligne d'injection 23 où se fait le mélange gazeux NO/N₂/O₂. Les mesures opérées par le capteur de débit 25 sont fournies aux moyens de pilotage de l'appareil 1 de délivrance de NO 1 où elles sont traitées et utilisées pour contrôler le débit de gaz à base de NO délivré, e.g. le mélange NO/N₂.

En effet, connaître le débit de gaz à base d'oxygène permet de réguler plus efficacement la délivrance du flux de NO (i.e. N₂/O₂) par le dispositif de délivrance de NO 1 grâce aux mesures de débit opérées par le capteur de débit 25 qui sont retournées, via la ligne de mesure de débit 26, aux moyens de pilotage de l'appareil de délivrance de NO 1.

Réguler le débit de NO (i.e. N₂/O₂) permet de fournir une proportion ou quantité adéquate de NO permettant d'obtenir, après mélange avec le flux de gaz à base d'oxygène provenant du ventilateur 50, la posologie en NO souhaitée dans le mélange final administré au patient, par exemple la concentration de NO mémorisée (NO_{mém}) s'affichant par défaut lors de la mise en service de l'appareil 1 comme expliqué ci-après.

Plus précisément, comme schématisé en Fig. 2, l'appareil de fourniture de NO 1 comprend, de manière classique, un boitier rigide, par exemple en polymère, traversé par un (ou des) passage de gaz interne 6, tel un conduit de gaz ou analogue, pour acheminer le flux de NO/N₂ amené par la (ou les) ligne d'amenée de gaz 12 qui est alimentée par les bouteilles de mélange de NO/N₂ 10.

Le passage de gaz interne 6 relie fluidiquement l'entrée (ou les entrées) de gaz 2 de l'appareil de fourniture de NO 1 à la ligne d'injection 23, via une sortie de NO 5 de l'appareil 1, de manière à convoyer le flux gazeux à base de NO entre eux. Des moyens à valve 7, i.e. un (ou des) dispositif à valve(s), par exemple une ou plusieurs électrovannes agencées en parallèle, préférentiellement une ou des (électro)vannes proportionnelles, sont agencés sur le passage de gaz interne 6 pour contrôler le flux gazeux qui y circule en direction de la sortie de gaz 5 alimentant la ligne d'injection 23.

Les moyens à valve 7 sont commandés par des moyens de pilotage 8, i.e. un (ou des) dispositif de pilotage, aussi appelé contrôleur ou électronique de commande, agencés dans le boitier de l'appareil de fourniture de NO 1, typiquement une carte électronique comprenant un (ou plusieurs) microprocesseur(s) 9, de préférence un (ou des) microcontrôleur, mettant en œuvre un ou des algorithmes. Ils peuvent comprendre d'autres éléments, comme un (ou des) mémoire informatique, telle une mémoire flash (non montrée).

Les moyens de pilotage 8 permettent notamment d'ajuster ou contrôler le débit de gaz à base de NO en pilotant les moyens à valve, typiquement d'ouvrir ou fermer tout ou partie de la ou des vannes, pour obtenir un débit de gaz déterminé qui est calculé par les moyens de pilotage 8 à partir d'une valeur de dose de NO souhaitée et en fonction du débit de gaz à base d'oxygène (i.e. air ou, N₂/O₂) provenant du ventilateur 50 et mesuré par le capteur de débit 25 qui est agencé sur la branche inspiratoire 21 et est relié au dispositif de fourniture de NO 1, par la ligne de mesure de débit 26, comme déjà expliqué.

Le passage de gaz interne 6 de l'appareil de fourniture de NO 1 peut aussi comprendre un (ou des) débitmètre (non montré) et/ou un régulateur de pression, tel un détendeur, (non montré) agencé en amont et/ou en aval des moyens à valve 7, pour déterminer le débit de gaz à base de NO circulant dans l'appareil de fourniture de NO 1. Le débitmètre peut être du type à différentiel de pression, à fil chaud ou autre. Il coopère avec les moyens de pilotage pour leur fournir, là encore, des mesures de débit du flux de NO/N₂, lesquelles mesures sont traitées par les moyens de pilotage 8 afin d'assurer une délivrance efficace de NO en fonction notamment du débit de gaz à base d'O₂ fourni par le ventilateur médical 50.

Habituellement, l'appareil de fourniture de NO 1 comprend aussi une interface graphique utilisateur ou IGU comprenant un afficheur graphique 4, de préférence un écran tactile, c'est-à-dire à dalle tactile, servant à afficher différentes informations ou données, icones, courbes, alarmes..., ainsi que des touches de sélection virtuelles et/ou des pavés ou fenêtres, servant notamment à opérer des choix, des sélections ou encore à entrer des informations, telles des valeurs désirées (e.g. débit, dosage de NO...), ou toute autre information ou donnée utile au personnel soignant. De préférence, l'affichage est en couleurs mais il peut se faire aussi en noir et blanc.

Les moyens de pilotage 8 de l'appareil de fourniture de NO 1 comprennent classiquement une carte de commande électronique et une unité de contrôle à microprocesseur 9, typiquement un microcontrôleur ou analogue. Les moyens de pilotage 8 permettent de contrôler ou commander tous les éléments électromécaniques de l'appareil 1. Plus précisément, la carte de commande intègre préférentiellement l'unité de contrôle et est configurée pour commander et par ailleurs analyser les signaux provenant des différents composants, tels que les capteurs...

L'alimentation électrique de l'appareil de fourniture de NO 1, en particulier des composants nécessitant du courant électrique pour fonctionner, tels les moyens de pilotage, l'afficheur graphique 4..., est assurée classiquement par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant. L'alimentation électrique du ventilateur médical 50 est assurée de façon analogue, notamment par une liaison au courant du secteur ou une batterie interne.

Enfin l'installation 100 comprend aussi une ligne de prélèvement de gaz 60 qui relie fluidiquement la branche inspiratoire 21 à l'appareil de fourniture de NO 1. Elle vient se raccorder fluidiquement (en 61) à la ligne d'alimentation en gaz 21, entre l'humidificateur 30 et la pièce de jonction 25, i.e. pièce en Y, typiquement à proximité immédiate de la pièce de jonction 25, et par ailleurs à un port d'entrée 62 du dispositif de fourniture de NO 1, par exemple un port 62 porté par un connecteur, raccord ou analogue, permettant le raccordement de la ligne de prélèvement de gaz 60, tel un tuyau flexible ou analogue, à une ligne d'analyse de gaz 111 munie de capteurs 112 d'un analyseur de gaz interne 110. Les échantillons de gaz prélevés dans la branche inspiratoire 21 sont convoyés jusqu'au dispositif de fourniture de NO 1 où ils sont analysés dans l'analyseur de gaz interne 110, typiquement un ou des capteurs 112 de NO₂, de NO et d'O₂, telles des cellules électrochimiques par exemple, reliés électriquement aux moyens de pilotage 8, afin de vérifier la conformité des échantillons gazeux analysés.

En effet, il convient de vérifier, pour des raisons de sécurité évidentes, que la composition du gaz final est conforme à celle du mélange gazeux NO/N₂/O₂ souhaité devant être administré au patient, notamment pour s'assurer qu'il ne contient pas de quantité excessive d'espèces NO₂ toxiques, que sa teneur en oxygène n'est pas hypoxique, qu'il ne contient pas une teneur en NO₂ trop élevée et que sa teneur en NO correspond à la posologie souhaitée, i.e. dose de NO à administrer par inhalation qui est habituellement choisie par le personnel soignant, i.e. médecin ou analogue.

Comme déjà expliqué, la dose de NO de départ est habituellement choisie, i.e. réglée ou sélectionnée, par le personnel médical au niveau du dispositif de fourniture de NO utilisé, après mise en route du dispositif de fourniture de NO. Ainsi, lorsqu'un dispositif de fourniture de NO est mis en fonction, c'est-à-dire « allumé » ou mis en service, l'afficheur 4 affiche une teneur ou dose de NO égale à 0 ppm et le personnel soignant doit alors fixer la dose de NO dite de « départ » ou de « démarrage » qui est celle souhaitée en début de traitement.

Autrement dit, lors de sa mise en route, un appareil de fourniture de NO 1 selon l'art antérieur affiche une dose dite « de départ » nulle (i.e. 0 ppm), c'est-à-dire une concentration de NO égale à 0 ppm, et le personnel soignant fixe une dose « de départ » souhaitée, par exemple 5, 10, 15 ou 20 ppm, en intervenant par exemple au niveau de l'interface utilisateur de l'appareil pour entrer la dose ou la sélectionner dans un menu affichant différents choix de doses possibles, ou analogue.

On comprend dès lors que des erreurs de dosage de NO peuvent parfois survenir résultant d'erreurs de réglage ou de sélection de dose de départ. En effet, le personnel soignant doit souvent agir dans l'urgence, lors d'un démarrage de traitement par NO chez un patient, par exemple un nouveau-né hypoxique, et peut dès lors se tromper en entrant ou sélectionnant la dose de NO à administrer au départ.

Or, on comprend aisément que toute erreur de dose, i.e. un sur- ou sous-dosage, peut avoir de graves conséquences pour le patient et ce, d'autant plus, que les personnes ayant besoin d'un traitement par iNO sont souvent fragiles et/ou dans un état médical grave, comme par exemple les nouveau-nés en hypoxémie sévère.

Selon l'invention, le dispositif 1 de fourniture de NO 1 a été modifié pour diminuer le risque de voir le patient recevoir une concentration ou dose non-adaptée, donc d'accroître la sécurité d'un traitement par NO en évitant les erreurs de réglage de dose de NO pouvant se produire en début de traitement, c'est-à-dire les erreurs liées à la fixation de la dose de NO de « départ », c'est-à-dire juste avant démarrage de l'administration du NO au patient, c'est-à-dire un début de traitement du patient.

Pour ce faire, selon l'invention, le dispositif 1 de fourniture de NO 1 est doté de moyens de mémorisation 70 configurés pour mémoriser une dose non-nulle de NO préfixée (NO_{mém}), c'est-à-dire une dose dotée et décidée à l'avance, typiquement une dose comprise entre 1 et 40 ppm, par exemple une dose de 10, 15, 20 ppm ou autre, qui sera la dose dite de départ.

Par ailleurs, les moyens de pilotage 8 incluant le microprocesseur 9, qui coopèrent avec les moyens de mémorisation 70, sont configurés pour commander un affichage (en 71) sur l'afficheur graphique 4 de la dose de NO préfixée (NO_{mém}) mémorisée en tant que dose de départ. Les moyens de mémorisation 70, par exemple une mémoire flash ou analogue, peuvent être agencés sur la carte électronique portant le microprocesseur 9 et reliés à celui-ci.

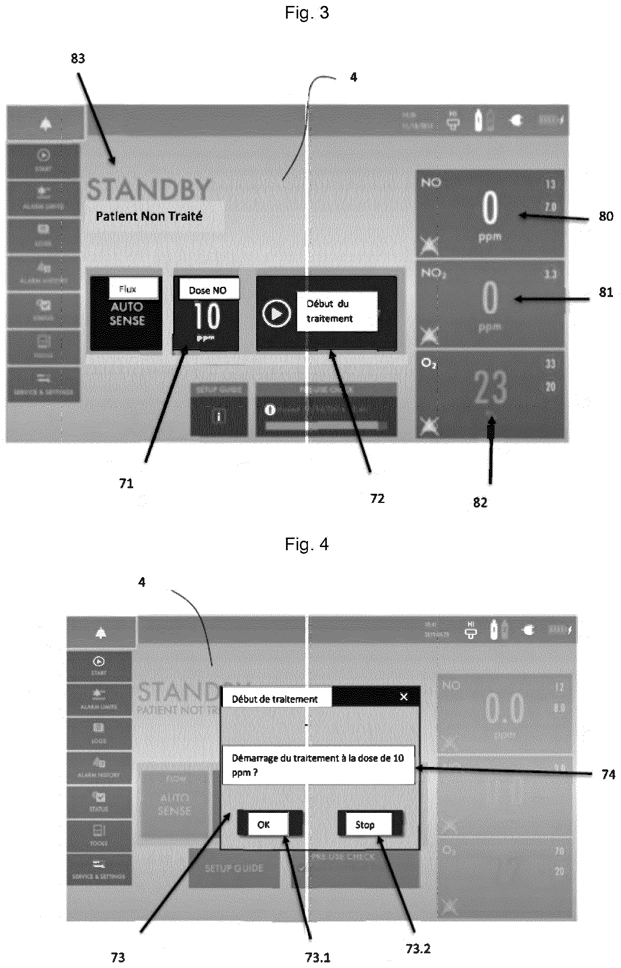

Autrement dit, les moyens de pilotage 8, typiquement le microprocesseur 9, pilotent ou commandent l'afficheur graphique 4 pour y réaliser un affichage par défaut de la dose de NO préfixée (NO_{mém}) mémorisée, dès mise en route de l'appareil 1, c'est-à-dire dès sa mise en service, au sein d'un espace dédié de l'écran 4, typiquement une fenêtre d'affichage 71 ou analogue. Par exemple, l'afficheur graphique 4 peut afficher (en 71) une valeur de dose de départ de « 10 ppm », comme illustré en Fig. 3.

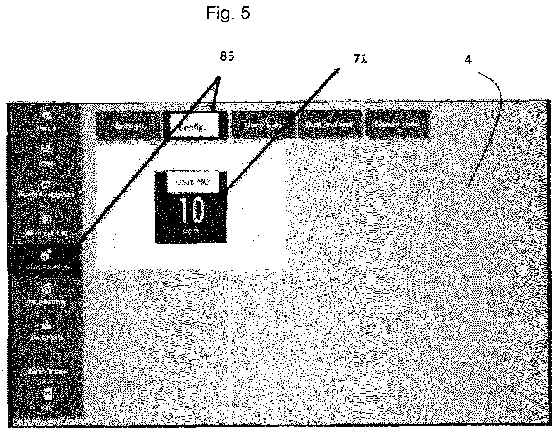

La dose de départ mémorisée peut être fixée une fois pour toute par le personnel soignant, par exemple par un membre de l'équipe de soin désigné par le Service hospitalier et de préférence en présence d'une personne certifiée par le fabricant de l'appareil, via un menu et/ou une ou des touches de configuration 85 de l'appareil 1 par exemple, comme schématisé en Fig. 5, et enregistrée ensuite par les moyens de mémorisation 70, par exemple une mémoire flash ou analogue. Cette dose pourra éventuellement être modifiée par la suite, via le menu de configuration et/ou la ou les touches de configuration 85, si le personnel soignant le souhaite et la nouvelle dose de départ sera alors mémorisée de la même manière.

Par ailleurs, l'appareil 1 comprend une touche 72 de début de traitement, actionnable par l'utilisateur, dont l'actionnement engendre une fourniture du flux de gaz contenant du NO, c'est-à-dire un débit de NO contrôle, pour obtenir la dose de NO préfixée mémorisée (NO_{mém}) dans le mélange final administré par inhalation au patient.

Avantageusement, l'afficheur graphique 4 est à dalle tactile, typiquement un écran en couleurs tactile *(touch pad* en anglais), et la touche de début de traitement 72, c'est-à-dire de validation de traitement, est une touche virtuelle affichée sur l'afficheur graphique 4, c'est-à-dire une touche tactile affichée sur l'écran tactile, comme illustré sur Fig. 3.

Lorsqu'un personnel soignant appuie avec son doigt, son index par exemple, sur la touche 72 de validation de début de traitement, cet appui va être reconnu par la dalle tactile et un signal correspondant va être transmis aux moyens de pilotage 8, typiquement au microprocesseur 9, qui vont alors commander les moyens à valve notamment pour débuter un traitement par iNO avec envoi d'un flux de NO dans la branche inspiratoire 21 du circuit patient 20, où il se mélange (en 24) au flux de gaz contenant l'oxygène provenant du ventilateur 50, tel de l'air ou un mélange O₂/N₂, et ainsi obtenir le mélange gazeux final souhaité contenant la posologie en NO désirée, à savoir la dose de NO préfixée mémorisée (NO_{mém}) de 10 ppm ici au début du traitement.

Bien entendu, cette dose peut être modifiée par la suite en fonction de la réponse du patient à son traitement par iNO, c'est-à-dire augmentée ou diminuée par le personnel soignant. Ceci peut se faire à tout moment, avant le démarrage du traitement et, par la suite, pendant toute la durée du traitement...

Dans l'exemple de Fig. 3, on voit comment peut se présenter l'écran 4 après sa mise en route par le personnel soignant mais avant toute administration de NO. La dose de NO préfixée mémorisée (NO_{mém}), à savoir 10 ppm ici, s'affiche par défaut dans la fenêtre d'affichage 71 et la touche 72 de validation de début de traitement est aussi affichée, juste à côté de la fenêtre d'affichage 71.

On voit sur Fig. 3 que, malgré l'affichage de la dose de départ de 10 ppm, l'administration de NO n'a pas encore débutée, c'est-à-dire que le dispositif 1 n'injecte pas encore de flux de NO, typiquement de mélange NO/N₂ à débit contrôlé, dans la branche inspiratoire 21 du circuit patient 20, étant donné que l'afficheur graphique 4 affiche par ailleurs des teneurs en NO et NO₂ nulles (i.e. 0 ppm) dans des fenêtres dédiées 80, 81. Par contre, on voit que le ventilateur 50 délivre déjà le flux de gaz contenant de l'oxygène (>20% vol), puisqu'une fenêtre 82 dédiée affiche une teneur en oxygène (O₂) de 23% ici. Ces concentrations en NO, O₂ et NO₂ sont déterminées par l'analyseur de gaz interne 110, à savoir la ligne d'analyse de gaz 111 munie de capteurs 112 de NO₂, de NO et d'O₂, telles des cellules électrochimiques, reliés électriquement aux moyens de pilotage 8, comme expliqué ci-avant.

Le fait que le traitement n'ait pas encore commencé est d'ailleurs confirmé par un affichage supplémentaire d'une information relative au traitement (i.e. en cours ou non) destinée au personnel soignant, dans un espace dédié 83 de l'écran 4, à savoir ici l'information suivante : STAND-BY (attente) - Patient Non Traité, ou une information similaire.

Bien entendu, l'information relative au traitement change dès appui du personnel patient sur la touche de début de traitement 72, c'est-à-dire est réactualisée. L'écran 4 peut alors afficher une information nouvelle du type : TRAITEMENT EN COURS - Patient Traité, ou analogue.

Le bon déroulement du traitement peut ensuite être vérifié par le personnel soignant en consultant notamment les fenêtres dédiées 80, 81, 82 qui affichent alors des valeurs de NO et O2 non nulles. La teneur en NO₂ doit être quant à elle aussi faible que possible, préférentiellement (quasi-)nulle, i.e. d'environ 0 ppm, car les espèces NO₂ (qui résultent d'une oxydation d'une faible partie du NO par de l'oxygène) sont toxiques et doivent être minimisées/évitées.

Selon un mode de réalisation avantageux, les moyens de pilotage 8 sont en outre configurés pour commander un affichage sur l'afficheur graphique 4 d'une (ou plusieurs) touche ou fenêtre de confirmation 73, c'est-à-dire d'une (ou des) autre touche virtuelle ou analogue, en réponse à un actionnement par l'utilisateur de la touche de début de traitement 72, comme illustré en Fig. 4.

Cette (ou ces) touche ou fenêtre de confirmation 73 permet au personnel soignant de confirmer ou annuler un début de traitement du patient avec démarrage à la dose de NO préfixée mémorisée (NO_{mém}), par exemple de 10 ppm dans l'exemple de Fig. 3.

Pour ce faire, selon le mode de réalisation, on peut prévoir soit deux touches de confirmation 73 distinctes, i.e. juxtaposées, ou, comme illustré en Fig. 4, une fenêtre de confirmation 73 divisée en deux zones distinctes juxtaposées 73.1, 73.2 :
- dont l'une 73.1 (i.e. première zone) permet de confirmer la dose de départ proposée, i.e. 10 ppm ici, et lancer le traitement, par exemple une touche ou zone 73.1 appelée « OK », « GO » ou « Début » ou tout terme similaire ; et
- dont l'autre 3.2 (i.e. seconde zone) permet d'annuler ou stopper tout démarrage de traitement avec fourniture de NO, par exemple une touche ou zone 73.2 appelée « Stop », « Abandon » ou « Arrêt » ou tout terme similaire.

La confirmation ou l'annulation (i.e. non-confirmation) se fait préférentiellement par appui du doigt (e.g. de l'index) par un personnel soignant sur l'une ou l'autre de ces zones ou touches distinctes 73.1, 73.2.

Comme illustré en Fig. 4, on peut prévoir que l'écran ou afficheur graphique 4 affiche, au premier plan, une fenêtre de confirmation 73 venant se superposer à la fenêtre d'affichage 71 de dose de NO de départ et à la touche de validation de début de traitement 72, en les masquant partiellement ou totalement. Le second plan de l'écran 4 peut alors être grisé.

De plus, l'afficheur graphique 4 peut être aussi configuré pour afficher simultanément une phrase ou autre (en 73) rappelant à l'utilisateur, ce qu'il doit confirmer ou infirmer, à savoir ici : *Démarrage du traitement à la dose de 10 ppm ?* ou d'autres informations.

Grâce à l'invention, aucune erreur de dose n'est possible au démarrage de l'appareil 1 car la dose de NO mémorisée est proposée, c'est-à-dire s'affiche par défaut (en 71) sur l'afficheur graphique 4, dès la mise en route de l'appareil 1, donc avant toute fourniture de NO à la branche inspiratoire 21 du circuit patient 20, donc au patient. La fourniture de NO ne débute ensuite qu'après validation de cette NO proposée par le personnel soignant, voire même uniquement après confirmation du traitement via la (ou les) touche ou fenêtre de confirmation 73.

## Revendications

1. Appareil de fourniture (1) d'un gaz contenant du NO comprenant :
- au moins un passage interne (6) pour acheminer un flux de gaz contenant du NO,
- des moyens à valve (7) agencés sur ledit au moins un passage interne,
- un afficheur graphique (4), et
- des moyens de pilotage (8) à microprocesseur (9) coopérant au moins avec les moyens à valve (7) pour contrôler la fourniture de gaz,
**caractérisé en ce que** :
- il comprend en outre des moyens de mémorisation (70) pour mémoriser une dose non-nulle de NO préfixée (NO_{mém}) correspondant à une concentration de NO dite de départ à administrer par inhalation à un patient au démarrage d'un traitement par NO inhalé,
- les moyens de pilotage (8) sont configurés pour commander un affichage sur l'afficheur graphique (4) de la dose de NO préfixée (NO_{mém}) ayant été mémorisée, et
- l'afficheur graphique (4) est configuré pour opérer, dès une mise en route ou en service de l'appareil et avant tout démarrage du traitement par NO inhalé, un affichage par défaut (en 71) de la dose de NO préfixée (NO_{mém}) ayant été mémorisée.

2. Appareil selon la revendication 1, **caractérisé en ce qu'**il comprend par ailleurs une touche de début de traitement (72), actionnable par l'utilisateur, dont l'actionnement engendre une fourniture du flux de gaz contenant du NO pour obtenir la dose de NO préfixée mémorisée (NO_{mém}).

3. Appareil selon les revendications 1 et 2, **caractérisé en ce que** l'afficheur graphique (4) est à dalle tactile et la touche de début de traitement (72) est une touche virtuelle affichée sur l'afficheur graphique (4).

4. Appareil selon l'une des revendications 2 ou 3, **caractérisé en ce que** les moyens de pilotage (8) sont en outre configurés pour commander un affichage sur l'afficheur graphique (4) d'au moins une touche ou fenêtre de confirmation (73) en réponse à un actionnement par l'utilisateur de la touche de début de traitement (72).

5. Appareil selon l'une des revendications 2 ou 3, **caractérisé en ce que** les moyens de pilotage (8) sont configurés pour commander les moyens à valve (7) pour fournir un débit donné de gaz contenant du NO permettant d'obtenir la dose de NO préfixée mémorisée (NO_{mém}), en réponse à un actionnement par l'utilisateur d'au moins la touche de début de traitement (72), en particulier d'au moins la touche de début de traitement (72) virtuelle affichée sur l'afficheur graphique (4).

6. Appareil selon les revendications 2 et 4, **caractérisé en ce que** les moyens de pilotage (8) sont configurés pour commander les moyens à valve (7) pour fournir un débit donné de gaz contenant du NO permettant d'obtenir la dose de NO préfixée mémorisée (NO_{mém}), en réponse à des actionnements successifs par l'utilisateur de la touche de début de traitement (72) puis de ladite au moins une touche ou fenêtre de confirmation (73).

7. Appareil selon à la revendication 6, **caractérisé en ce que** le ou les actionnements par l'utilisateur de la touche de début de traitement (72) et/ou de ladite au moins une touche ou fenêtre de confirmation (73) se font par appui digital de l'utilisateur.

8. Appareil selon la revendication 1, **caractérisé en ce que** les moyens de mémorisation (70) sont configurés pour mémoriser une dose de NO préfixée (NO_{mém}) comprise entre 1 et 40 ppmv, de préférence entre 5 et 20 ppmv.

9. Appareil selon la revendication 1, **caractérisé en ce que** les moyens de pilotage (8) sont configurés pour commander un affichage de la dose de NO préfixée (NO_{mém}) ayant été mémorisée, dès mise en service de l'appareil (1) mais avant tout début de traitement.

10. Appareil selon la revendication 8, **caractérisé en ce que** les moyens de mémorisation (70) sont configurés pour mémoriser une dose de NO préfixée (NO_{mém}) de 5, 10, 15 ou 20 ppmv.

11. Appareil selon l'une des revendications 1 ou 2, **caractérisé en ce que** l'afficheur graphique (4) est configuré pour afficher une première touche ou zone de confirmation dont la sélection par l'utilisateur, par appui digital, permet de confirmer la dose de départ proposée et lancer le traitement avec fourniture de NO.

12. Appareil selon l'une des revendications 2 ou 11, **caractérisé en ce que** l'afficheur graphique (4) est configuré pour afficher une seconde touche ou zone de confirmation dont la sélection par l'utilisateur, par appui digital, permet d'annuler ou stopper tout démarrage de traitement avec fourniture de NO.

13. Appareil selon les revendications 11 et 12, **caractérisé en ce que** l'afficheur graphique (4) est configuré pour afficher la seconde touche ou zone de confirmation en réponse à un appui par l'utilisateur sur la première touche ou zone de confirmation.

14. Installation d'administration de gaz (100) à un patient, i.e. d'un gaz contenant du NO, comprenant :
- au moins une source de gaz (10) contenant du NO gazeux, en particulier un mélange gazeux NO/N₂,
- un appareil de fourniture de gaz (1) selon l'une des revendications précédentes, alimenté en gaz contenant du NO par ladite au moins une source de gaz (10), tel le mélange gazeux NO/N₂,
- un ventilateur médical (50) pour fournir un gaz contenant de l'oxygène, tel de l'air ou un mélange gazeux O₂/N₂, et
- une ligne d'alimentation (20, 21) alimentée par l'appareil de fourniture de gaz (1) en gaz contenant du NO, tel le mélange gazeux NO/N₂, et par le ventilateur médical (50) en gaz contenant de l'oxygène, tel de l'air ou le mélange O₂/N₂.

15. Installation selon la revendication 14, **caractérisé en ce que** ladite au moins une source de gaz (10) contient un mélange gazeux NO/N₂ contenant moins de 2000 ppmv de NO, le reste étant de l'azote.

## Patentansprüche

1. Vorrichtung zum Zuführen (1) eines NO-haltigen Gases, umfassend:
- mindestens einen inneren Durchgang (6) zum Leiten eines Stroms von NO-haltigem Gas,
- Ventilmittel (7), die an dem besagten mindestens einen inneren Durchgang angeordnet sind,
- eine grafische Anzeige (4), und
- Steuerungsmittel (8) mit einem Mikroprozessor (9), die mindestens mit den Ventilmitteln (7) zusammenwirken, um die Zufuhr von Gas zu steuern,
**dadurch gekennzeichnet, dass**:
- sie ferner Speichermittel (70) zum Speichern einer voreingestellten Nicht-Null-Dosis von NO (NOmem) umfasst, die einer als Ausgangskonzentration bezeichneten NO-Konzentration entspricht, die einem Patienten zu Beginn einer Behandlung mit inhaliertem NO durch Inhalation zu verabreichen ist,
- die Steuerungsmittel (8) so konfiguriert sind, dass sie eine Anzeige der voreingestellten NO-Dosis (NOmem), die gespeichert wurde, auf der grafischen Anzeige (4) ansteuern, und
- die grafische Anzeige (4) so konfiguriert ist, dass sie bei Inbetriebnahme oder Indienststellung der Vorrichtung und vor jedem Beginn der Behandlung mit inhaliertem NO eine Standardanzeige (bei 71) der voreingestellten NO-Dosis (NOmem), die gespeichert wurde, durchführt.

2. Vorrichtung nach Anspruch 1, **dadurch gekennzeichnet, dass** sie darüber hinaus eine Behandlungsstarttaste (72) umfasst, die vom Benutzer betätigt werden kann und deren Betätigung eine Zufuhr des Stroms von NO-haltigem Gas bewirkt, um die voreingestellte gespeicherte NO-Dosis (NOmem) zu erhalten.

3. Vorrichtung nach den Ansprüchen 1 und 2, **dadurch gekennzeichnet, dass** die grafische Anzeige (4) ein Touchscreen ist und die Behandlungsstarttaste (72) eine virtuelle Taste ist, die auf der grafischen Anzeige (4) angezeigt wird.

4. Vorrichtung nach einem der Ansprüche 2 oder 3, **dadurch gekennzeichnet, dass** die Steuerungsmittel (8) ferner so konfiguriert sind, dass sie als Reaktion auf eine Betätigung der Behandlungsstarttaste (72) durch den Benutzer eine Anzeige von mindestens einer Bestätigungstaste oder einem Bestätigungsfenster (73) auf der grafischen Anzeige (4) ansteuern.

5. Vorrichtung nach einem der Ansprüche 2 oder 3, **dadurch gekennzeichnet, dass** die Steuerungsmittel (8) so konfiguriert sind, dass sie die Ventilmittel (7) ansteuern, um einen gegebenen Durchfluss von NO-haltigem Gas zu liefern, der es ermöglicht, die voreingestellte gespeicherte NO-Dosis (NOmem) zu erhalten, als Reaktion auf eine Betätigung durch den Benutzer von mindestens der Behandlungsstarttaste (72), insbesondere von mindestens der virtuellen Behandlungsstarttaste (72), die auf der grafischen Anzeige (4) angezeigt wird.

6. Vorrichtung nach den Ansprüchen 2 und 4, **dadurch gekennzeichnet, dass** die Steuerungsmittel (8) so konfiguriert sind, dass sie die Ventilmittel (7) ansteuern, um einen gegebenen Durchfluss von NO-haltigem Gas zu liefern, der es ermöglicht, die voreingestellte gespeicherte NO-Dosis (NOmem) zu erhalten, als Reaktion auf aufeinanderfolgende Betätigungen durch den Benutzer der Behandlungsstarttaste (72) und dann der besagten mindestens einen Bestätigungstaste oder des Bestätigungsfensters (73).

7. Vorrichtung nach Anspruch 6, **dadurch gekennzeichnet, dass** die Betätigung oder die Betätigungen durch den Benutzer der Behandlungsstarttaste (72) und/oder der besagten mindestens einen Bestätigungstaste oder des Bestätigungsfensters (73) durch digitalen Druck des Benutzers erfolgen.

8. Vorrichtung nach Anspruch 1, **dadurch gekennzeichnet, dass** die Speichermittel (70) so konfiguriert sind, dass sie eine voreingestellte NO-Dosis (NOmem) zwischen 1 und 40 ppmv, vorzugsweise zwischen 5 und 20 ppmv, speichern.

9. Vorrichtung nach Anspruch 1, **dadurch gekennzeichnet, dass** die Steuerungsmittel (8) so konfiguriert sind, dass sie eine Anzeige der voreingestellten NO-Dosis (NOmem), die gespeichert wurde, bei der Indienststellung der Vorrichtung (1), aber vor jedem Behandlungsbeginn, ansteuern.

10. Vorrichtung nach Anspruch 8, **dadurch gekennzeichnet, dass** die Speichermittel (70) so konfiguriert sind, dass sie eine voreingestellte NO-Dosis (NOmem) von 5, 10, 15 oder 20 ppmv speichern.

11. Vorrichtung nach einem der Ansprüche 1 oder 2, **dadurch gekennzeichnet, dass** die grafische Anzeige (4) so konfiguriert ist, dass sie eine erste Bestätigungstaste oder -zone anzeigt, deren Auswahl durch den Benutzer durch digitalen Druck es ermöglicht, die vorgeschlagene Startdosis zu bestätigen und die Behandlung mit NO-Zufuhr zu starten.

12. Vorrichtung nach einem der Ansprüche 2 oder 11, **dadurch gekennzeichnet, dass** die grafische Anzeige (4) so konfiguriert ist, dass sie eine zweite Bestätigungstaste oder -zone anzeigt, deren Auswahl durch den Benutzer durch digitalen Druck es ermöglicht, jeden Behandlungsstart mit NO-Zufuhr abzubrechen oder zu stoppen.

13. Vorrichtung nach den Ansprüchen 11 und 12, **dadurch gekennzeichnet, dass** die grafische Anzeige (4) so konfiguriert ist, dass sie die zweite Bestätigungstaste oder -zone als Reaktion auf einen Druck des Benutzers auf die erste Bestätigungstaste oder -zone anzeigt.

14. Anlage zur Verabreichung von Gas (100) an einen Patienten, d.h. eines NO-haltigen Gases, umfassend:
- mindestens eine Gasquelle (10), die gasförmiges NO enthält, insbesondere ein gasförmiges NO/N2-Gemisch,
- eine Vorrichtung zum Zuführen von Gas (1) nach einem der vorhergehenden Ansprüche, die mit NO-haltigem Gas von der besagten mindestens einen Gasquelle (10) versorgt wird, wie z.B. das gasförmige NO/N2-Gemisch,
- ein medizinisches Beatmungsgerät (50) zum Zuführen eines sauerstoffhaltigen Gases, wie Luft oder ein gasförmiges O2/N2-Gemisch, und
- eine Zufuhrleitung (20, 21), die von der Vorrichtung zum Zuführen von Gas (1) mit NO-haltigem Gas, wie z.B. dem gasförmigen NO/N2-Gemisch, und von dem medizinischen Beatmungsgerät (50) mit sauerstoffhaltigem Gas, wie Luft oder dem O2/N2-Gemisch, versorgt wird.

15. Anlage nach Anspruch 14, **dadurch gekennzeichnet, dass** die besagte mindestens eine Gasquelle (10) ein gasförmiges NO/N2-Gemisch enthält, das weniger als 2000 ppmv NO enthält, wobei der Rest Stickstoff ist.

## Claims

1. An apparatus for supplying (1) a gas containing NO comprising:
- at least one internal passage (6) for conveying a flow of gas containing NO,
- valve means (7) arranged on said at least one internal passage,
- a graphical display (4), and
- piloting means (8) with a microprocessor (9) cooperating with at least the valve means (7) to control the supply of gas,
**characterized in that**:
- it further comprises memorization means (70) for memorizing a non-zero preset dose of NO (NOmem) corresponding to a starting concentration of NO to be administered by inhalation to a patient at the start of a treatment with inhaled NO,
- the piloting means (8) are configured to command a display on the graphical display (4) of the preset dose of NO (NOmem) having been memorized, and
- the graphical display (4) is configured to operate, upon startup or putting into service of the apparatus and before any start of treatment with inhaled NO, a default display (at 71) of the preset dose of NO (NOmem) having been memorized.

2. The apparatus according to claim 1, **characterized in that** it further comprises a treatment start button (72), activatable by the user, the activation of which generates a supply of the flow of gas containing NO to obtain the preset memorized dose of NO (NOmem).

3. The apparatus according to claims 1 and 2, **characterized in that** the graphical display (4) is a touchscreen and the treatment start button (72) is a virtual button displayed on the graphical display (4).

4. The apparatus according to one of claims 2 or 3, **characterized in that** the piloting means (8) are further configured to command a display on the graphical display (4) of at least one confirmation button or window (73) in response to an activation by the user of the treatment start button (72).

5. The apparatus according to one of claims 2 or 3, **characterized in that** the piloting means (8) are configured to command the valve means (7) to supply a given flow rate of gas containing NO allowing to obtain the preset memorized dose of NO (NOmem), in response to an activation by the user of at least the treatment start button (72), in particular of at least the virtual treatment start button (72) displayed on the graphical display (4).

6. The apparatus according to claims 2 and 4, **characterized in that** the piloting means (8) are configured to command the valve means (7) to supply a given flow rate of gas containing NO allowing to obtain the preset memorized dose of NO (NOmem), in response to successive activations by the user of the treatment start button (72) then of said at least one confirmation button or window (73).

7. The apparatus according to claim 6, **characterized in that** the activation or activations by the user of the treatment start button (72) and/or of said at least one confirmation button or window (73) are performed by digital press by the user.

8. The apparatus according to claim 1, **characterized in that** the memorization means (70) are configured to memorize a preset dose of NO (NOmem) comprised between 1 and 40 ppmv, preferably between 5 and 20 ppmv.

9. The apparatus according to claim 1, **characterized in that** the piloting means (8) are configured to command a display of the preset dose of NO (NOmem) having been memorized, upon putting the apparatus (1) into service but before any start of treatment.

10. The apparatus according to claim 8, **characterized in that** the memorization means (70) are configured to memorize a preset dose of NO (NOmem) of 5, 10, 15 or 20 ppmv.

11. The apparatus according to one of claims 1 or 2, **characterized in that** the graphical display (4) is configured to display a first confirmation button or zone, the selection of which by the user, by digital press, allows to confirm the proposed starting dose and to launch the treatment with supply of NO.

12. The apparatus according to one of claims 2 or 11, **characterized in that** the graphical display (4) is configured to display a second confirmation button or zone, the selection of which by the user, by digital press, allows to cancel or stop any start of treatment with supply of NO.

13. The apparatus according to claims 11 and 12, **characterized in that** the graphical display (4) is configured to display the second confirmation button or zone in response to a press by the user on the first confirmation button or zone.

14. An installation for administering gas (100) to a patient, i.e., of a gas containing NO, comprising:
- at least one gas source (10) containing gaseous NO, in particular a gaseous NO/N2 mixture,
- an apparatus for supplying gas (1) according to any one of the preceding claims, supplied with gas containing NO by said at least one gas source (10), such as the gaseous NO/N2 mixture,
- a medical ventilator (50) for supplying a gas containing oxygen, such as air or a gaseous O2/N2 mixture, and
- a supply line (20, 21) supplied by the apparatus for supplying gas (1) with gas containing NO, such as the gaseous NO/N2 mixture, and by the medical ventilator (50) with gas containing oxygen, such as air or the O2/N2 mixture.

15. The installation according to claim 14, **characterized in that** said at least one gas source (10) contains a gaseous NO/N2 mixture containing less than 2000 ppmv of NO, the rest being nitrogen.
